(19) **Europäisches Patentamt European Patent Office Office européen des brevets**

(11) **EP 4 171 435 B1**

(12) **EUROPÄISCHE PATENTSCHRIFT**

(45) Veröffentlichungstag und Bekanntmachung des Hinweises auf die Patenterteilung:
**01.10.2025   Patentblatt 2025/40**

(21) Anmeldenummer: **21737331.5**

(22) Anmeldetag: **25.06.2021**

(51) Internationale Patentklassifikation (IPC):
*A61C 9/00* (2006.01)     *A61B 1/00* (2006.01)
*A61B 1/24* (2006.01)     *A61B 5/00* (2006.01)
*G06T 5/00* (2024.01)     *G01J 3/50* (2006.01)
*G01J 3/52* (2006.01)

(52) Gemeinsame Patentklassifikation (CPC):
**A61C 9/0053; A61B 1/00009; A61B 1/00194; A61B 1/24; A61B 5/0062; A61B 5/0088; A61B 5/1034; A61B 5/7246;** A61B 2576/02; G01J 3/508; G01J 3/524

(86) Internationale Anmeldenummer:
**PCT/EP2021/067560**

(87) Internationale Veröffentlichungsnummer:
**WO 2021/260199 (30.12.2021 Gazette 2021/52)**

(54) **VERFAHREN ZUR DIGITALEN ERFASSUNG EINER INTRAORALEN STRUKTUR UND SYSTEM ZUR DURCHFÜHRUNG DES VERFAHRENS**

METHOD FOR THE DIGITAL ACQUISITION OF AN INTRAORAL STRUCTURE AND SYSTEM FOR CARRYING OUT THE METHOD

PROCÉDÉ D'ACQUISITION NUMÉRIQUE D'UNE STRUCTURE INTRABUCCALE ET SYSTÈME POUR LA MISE EN OEUVRE DU PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **26.06.2020   DE 102020003856**

(43) Veröffentlichungstag der Anmeldung:
**03.05.2023   Patentblatt 2023/18**

(73) Patentinhaber: **Ivoclar Vivadent AG**
**9494 Schaan (LI)**

(72) Erfinder:
• **SCHWEIGER, Josef**
  **83346 Bergen (DE)**
• **GÜTH, Jan-Frederik**
  **81667 München (DE)**

(74) Vertreter: **Baldus, Oliver**
**Splanemann**
**Rumfordstrasse 7**
**80469 München (DE)**

(56) Entgegenhaltungen:
WO-A2-2019/220212     CN-A- 111 136 915
US-A1- 2008 094 631     US-A2- 2017 252 135

• **LV JINGQIN ET AL: "A Color Distance Model Based on Visual Recognition", MATHEMATICAL PROBLEMS IN ENGINEERING, vol. 2018, 1 January 2018 (2018-01-01), CH, pages 1 - 7, XP055842982, ISSN: 1024-123X, Retrieved from the Internet <URL:https://downloads.hindawi. com/journals/mpe/2018/4652526.pdf> DOI: 10.1155/2018/4652526**

**Beschreibung**

Die Erfindung bezieht sich auf ein Verfahren zur digitalen Erfassung einer intraoralen Struktur durch Abtastung der Struktur mit einem Intraoralscanner, dessen Abtastwerte die räumliche Lage der Abtaststellen darstellende Ortsinformationsdaten und Farbwerte der Abtaststellen darstellende Farbinformationsdaten enthalten, sowie auf ein System zur Durchführung des Verfahrens.

Die Druckschrift US 2017/252135 A2 betrifft eine Gingiva-Indexierungsvorrichtung mit verschiedenen, definierten roten Farbregionen (Rottönen) sowie ein Verfahren zur Erfassung der Gingiva-Situation. Ein Bereich der Gingiva-Situation wird dreidimensional digital erfasst. Der Bereich wird zusammen mit einer Farbgrau- und/oder einer Graukarte zur Kalibrierung gescannt.

Die Druckschrift US 2008/094631 A1 betrifft Geräte und Verfahren zum Messen optischer Eigenschaften wie Farbspektren, Lichtdurchlässigkeit, Glanz und anderer Eigenschaften von Objekten wie Zähnen. Ein Farbkalibrierungsdiagramm kann von einem Computer 384 verwendet werden, um die Farbdaten in einem aufgenommenen Bild auf echte oder bekannte Farbwerte zu "kalibrieren".

Die Erfindung, welche in den angehängten Ansprüchen 1 bis 11 definiert ist, bezweckt eine digitale dreidimensionale intraorale Erfassung von Zähnen, Gingiva oder Kieferbereichen mit geometriebezogener Farbinformation.

Die digitale Erfassung von intraoralen Strukturen mittels digitaler 3D-Scans gibt es seit nunmehr 33 Jahren im Dentalbereich. Bis vor wenigen Jahren beschränkte sich dabei der Intraoralscan auf die Messung und Ermittlung der Geometriedaten der Oberflächen, welche durch die in den Abtastwerten eines Intraoralscanners enthaltenen Ortsinformationsdaten dargestellt werden. Seit einigen Jahren gibt es nun auch Scanner- Anbieter, welche neben der eigentlichen Oberflächeninformation auch die Farbinformation mit erfassen und ausgeben. Die Besonderheit liegt darin, dass die Farbinformationen geometriebezogen sind. Dies bedeutet, dass jeder Stelle auf dem Oberflächendatensatz eine definierte Farbe zugeordnet ist. Es gibt verschiedene Dateiformate, welche beide Informationen (Geometrie, geometriebezogene Farbe) darstellen können, wie beispielsweise die Formate PLY, OBJ, VRML etc.

Derzeit gibt es keine Möglichkeit, die geometriebezogene Farbinformation des Intraoralscans farbfehlerfrei darzustellen. Die Geräte der unterschiedlichen Hersteller geben daher die Farbe sehr unterschiedlich aus. Der Wert der Farbinformation für anschließende Weiterbearbeitungsschritte ist daher dementsprechend gering oder die Farbinformationen sind gar wertlos.

Der Erfindung liegt die Aufgabe zugrunde, ein Verfahren der eingangs genannten Art dahingehend weiterzubilden, dass Farbfehler gescannter intraoraler Strukturen korrigiert werden können, sowie eine zur Durchführung des Verfahrens geeignete Farbreferenzvorlage und ein zur Durchführung des Verfahrens geeignetes System anzugeben.

Erfindungsgemäß wird diese Aufgabe hinsichtlich des Verfahrens dadurch gelöst, dass im Abtastbereich der Struktur eine Farbreferenzvorlage, die ein einem Referenzfarbwert eines Referenzfarbraums entsprechendes Referenzfarbfeld aufweist, angeordnet wird, und dass die abgetasteten Farbinformationsdaten entsprechend dem Abstand zwischen dem Referenzfarbwert und einem abgetasteten Farbwert des Referenzfarbfelds korrigiert werden.

Insbesondere wird dabei folgendermaßen vorgegangen:

Bevor mit dem dreidimensionalen Intraoralscan begonnen wird, wird der Scanner wie vom Hersteller vorgesehen kalibriert. Anschließend wird vor dem intraoralen Einscannen der intraoralen Struktur, insbesondere der Gingiva und der Zähne die Referenzvorlage, die beispielsweise als "Graukartenpunkt" ausgebildet ist, auf die Gingiva aufgebracht. Der Punkt hat beispielsweise eine graue Farbe, die exakt den Farbwert einer in der Fotografie zur Farbkalibrierung verwendeten Graukarte hat. Dieser "Graukartenpunkt" kann in verschiedenen geometrischen Formen gestaltet sein, wie beispielsweise eine flache zweidimensionale Scheibe, eine dreidimensional geformte Halbkugel etc. Der Punkt kann als konfektioniertes Produkt angeboten werden, wobei die zur Gingiva hin gerichtete Fläche mit einem Klebstoff versehen sein kann, sodass der "Graukartenpunkt" auf die Gingiva oder Zähne aufgeklebt werden kann. Nach dem Aufkleben des "Graukartenpunktes" werden die Kiefer (Zähne und Gingiva) mit dem 3D-Intraoralscanner eingescannt. Diese Aufnahme wird "Referenzaufnahme" genannt. Der verwendete Scanner muss die Möglichkeit anbieten, neben der dreidimensionalen Oberfläche auch die geometriebezogene Farbe erfassen zu können. Beim Scannen wird neben den Zähnen und der Gingiva auch der "Graukartenpunkt" mit gescannt. Somit ist die standardisierte Farbinformation der Graukarte in den geometriebezogenen Farbinformationen des 3D-Intraoralscans abgebildet. Weiterhin können auch mehr als 1 Farbreferenzpunkt auf die Gingiva oder die Zähne aufgeklebt werden, beispielsweise ein Punkt im Frontzahnbereich und je ein Punkt in den Quadranten des Seitenzahnbereiches. Damit können unterschiedliche externe Belichtungseinflüsse z.B. durch Umgebungslicht (Raumleuchten, Leuchte am Praxisstuhl, Tageslicht etc.) im Seitenzahn- und Frontzahnbereich kompensiert werden. Die nachfolgenden farblichen Korrekturen können entsprechend segmentweise erfolgen.

Optional kann anstelle des das Referenzfarbfeld aufweisenden "Graukartenpunktes" ein "Mehrfarbenpunkt" verwendet werden. Dieser Punkt enthält mindestens eine Farbe und kann eine unbegrenzte Anzahl von Farben enthalten, die jeweils exakt definiert sind. In einer in Fig, 1 dargestellten besonderen Ausführungsform enthält der Punkt (1) die Farben grau (3) (wie Graukartenpunkt), die Farbe weiß (2) und die Farbe schwarz (4). Auch dieser "Mehrfarbenpunkt" kann dreidimensional gestaltet sein, z.B. Halbkugel, Tetraeder, Pyramide etc. Durch die zusätzlichen Farben weiß und schwarz kann optional eine genauere Kalibrierung der Farbaufnahmen vorgenommen werden.

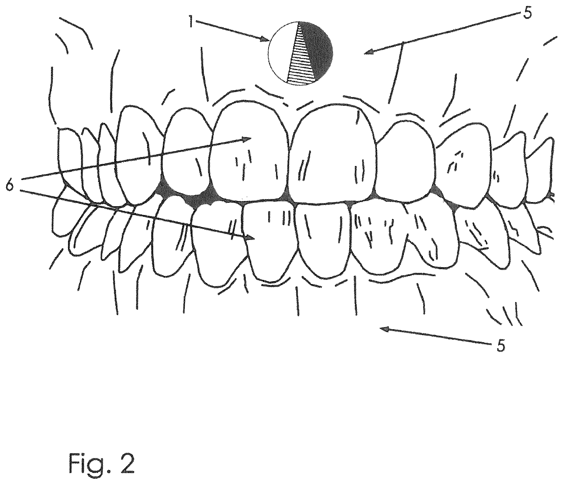

Fig. 2 zeigt die intraorale Ausgangssituation mit Gingiva (5) und Zahnreihen (6), in der dieser Graukartenpunkt auf die Gingiva aufgeklebt ist.

Mittels Software ist es möglich, die Farbinformationsdaten und die 3D- Oberflächeninformation, die durch die Ortsinformationsdaten dargestellt wird, zu separieren. Es entsteht ein zweidimensionales Bild der Farbinformation und der 3D- Datensatz des gescannten Kiefers mit Zähnen und Gingiva. Zusätzlich gibt es eine weitere Datei, welche die zweidimensionale pixelweise Farbinformation einem dreidimensionalen Punkt auf dem gescannten 3D-Oberflächendatensatz zuordnet (= "Mapping-Datei").

Da die zweidimensionale Farbinformation auch die Informationen des "Graukartenpunktes"/"Mehrfarbenpunktes" abbildet, ist es möglich, die Referenzaufnahme anhand des standardisierten Grauwertes/Schwarzwertes/Weißwertes zu korrigieren. Dazu kann beispielsweise die Software Adobe Photoshop oder Adobe Lightroom verwendet werden. Nach diesem Schritt ist die geometriebezogene Farbinformation ebenfalls korrigiert, sodass das Endresultat ein farbrichtiger 3D-Intraoral-Farbscan ist. Der Gesamtdatensatz wird aus zwei Einzeldatensätzen gebildet. Die Zuordnung der Farbinformation zum 3D-Oberflächendatensatz erfolgt anhand einer sogenannten "Mapping-Datei". Die Farbkorrektur des Datensatzes der Farbinformation aus dem intraoralen 3D-Scan kann mit gängigen Bildbearbeitungs-Programmen, wie beispielsweise Adobe Photoshop, erfolgen. Beispielsweise erfolgt die Korrektur der Farben mittels Tonwertkorrektur und Graukartenpunkt

Das farblich korrigierte Bild wird wieder in den Gesamtdatensatz des intraoralen 3D- Scans kopiert und das bisherige Bild damit ersetzt. Nun ordnet die "Mapping-Datei" die korrigierten Farbwerte wieder den 3D-Punkten des Oberflächenscans ohne Veränderung der Ausrichtung zu. Somit ist die farbliche Korrektur der geometriebezogenen Farbinformation finalisiert.

Anschließend kann der korrigierte Datensatz für die Herstellung, insbesondere additive Herstellung z.B. mittels Multimaterial-3D-Druck, eines physischen Farbmodells oder einer dentalen Restauration wie beispielsweise von Kronen, Brücken oder Prothesen verwendet werden. Neben der additiven Fertigung können auch Herstellungsverfahren wie beispielsweise Fräsen, Pressen, Gießen oder Tiefziehen für die Weiterverarbeitung der korrigierten Datensätze verwendet werden. So kann beispielsweise auf eine keramische Versorgung, welche mittels CAD/CAM-Technik subtraktiv hergestellt wurde, in einem nachfolgenden Schritt die Farbe mittels Druckkopf aufgetragen werden. In der Patentschrift DE 102006 061 893 B3 ist eine entsprechende Technologie beschrieben.

Die Weiterverarbeitung des korrigierten Datensatzes erfolgt insbesondere mit einer sogenannten Modelbuilder-Software. Aus den aufbereiteten Daten wird mittels additiver Verfahren ein realistisches und farbtreues 3D-Modell der Kiefersituation mittels Multimaterial 3D-Druck additiv hergestellt, das ein Meistermodell mit geometriebezogener Farbinformation (graphisches 3D-Modell) ist.

Die Detektion der abgetasteten Farbinformationen des Referenzfarbfelds, insbesondere des Graukartenpunktes/Mehrfarbenpunktes im dreidimensionalen gescannten Datensatz oder im ausgegebenen zweidimensionalen Bild kann neben der manuellen Detektion durch den Benutzer (im 3D-Scandatensatz oder im zweidimensionalen Bild) auch teilautomatisch oder vollautomatisch erfolgen. Bei der Erkennung der Farbinformation muss man zwischen dem flachen zweidimensionalen Graukartenpunkt/Mehrfarbenpunkt und dem dreidimensional geformten Graukartenpunkt/Mehrfarbenpunkt unterscheiden. Automatische Detektion des zweidimensional geformten Graukartenpunktes/Mehrfarbenpunktes

Erfindungsgemäß können verschiedene Farbmodelle Anwendung finden, wie beispielsweise RGB, CMYK, L* a* b*, CIE Lab und weitere. Bei diesen Farbmodellen werden den Einzelfarben der Farbmodelle Zahlenwerte zugeordnet, beispielsweise beim RGB-Farbmodell den Farben Rot, Grün und Blau. Die Zahlenwerte der Farben können dabei in der klassischen Darstellung Werte zwischen 0 und 1 haben In der computerorientierten Anwendung werden Ganzzahlen zwischen 0 und 255 abgespeichert. Beispielhaft sei hier der RGB-Farbraum dargestellt:

Der Farbe Weiß werden die Werte R 255, G 255, B 255, der Farbe Schwarz die R 0, G 0, B 0 und der Norm-Graukarte (18% Grau) die Werte R 128, G 128, B 128 zugeordnet.

Moderne computerorientierte Applikationen und Schnittstellen verwenden teilweise statt der 8 Bit vorzeichenlosen Ganzzahlen intern häufig Gleitkommazahlen, die einen größeren Wertebereich mit höherer Auflösung repräsentieren.

Um eine analoge Detektion des Graukartenpunktes/Mehrfarbenpunktes auszuführen, ist es absolut erforderlich, ein zweidimensionales Bild der Farbinformation zu haben. Allerdings zeigen die Daten verschiedener 3D-Intraoralscanner eine unterschiedliche Darstellung dieser Farbpunkte, sodass diese in analoger Weise schlecht bis gar nicht detektierbar sind. Zudem ist es auch möglich, dass die Farbinformation als reiner Zahlencode im entsprechenden gescannten Datensatz vorliegt, sodass auch hier eine Detektion durch den Anwender nur schwer oder gar nicht möglich ist. Daher ist es sinnvoll, einen automatisierten Algorithmus zur Erkennung des Graukartenpunktes/Mehrfarbenpunktes zu verwenden. Die vorliegende Erfindung behebt diesen Mangel in folgender Weise:

Die erfassten Zahlenwerte der Farbinformationen bilden einen n-dimensionalen Raum (z.B. dreidimensionaler Farbraum bei RGB-Farbmodell). Die Farbwerte des Graukartenpunktes/Mehrfarbenpunktes haben dabei in diesem Farbraum eine definierte Position/festen Wert. Mit Hilfe des erfindungsgemäßen Berechnungsalgorithmus werden alle erfassten Farbwerte der Abtaststellen mit den definierten Farbwerten des Referenzfarbfeldes verglichen und die Farbwerte selektiert, welche den definierten Farbwerten am nächsten kommen. Dieser kleinste Abstand wird nach dem Prinzip des "Euklidischen Abstandes" berechnet.

Der Abstand eines gemessenen Farbwertes (p) im n-dimensionalen Farbraum zum Referenzfarbwert des Graukartenpunktes/Mehrfarbenpunktes (q) wird wie folgt berechnet:

$$d(p, q) = \|q - p\|_2 = \sqrt{(q_1 - p_1)^2 + \cdots + (q_n - p_n)^2} = \sqrt{\sum_{i=1}^{n} (q_i - p_i)^2}$$

Folgende Werte des Graukartenpunktes/Mehrfarbenpunktes (q) werden beispielsweise im dreidimensionalen RGB-Farbraum angewendet:

Weiß (R255,G255,B255)
Schwarz (R0,G0,B0)
18% Grau (R128,G218,B218)

Durch Festlegung eines Maximalwertes (Schwellenwert) für den Abstand des gemessenen Farbwertes (p) im n-dimensionalen Farbraum zum Referenzfarbwert des Graukartenpunktes/Mehrfarbenpunktes (q) kann die Anzahl der ermittelten Messwerte individuell gesteuert werden.

Für alle pixelweise delektierten Abstände des Graukartenpunktes/Mehrfarbenpunktes wird ein Mittelwert/Medianwert gebildet werden, welcher anschließend als Korrekturwert für alle gemessenen Farbwerte verwendet wird.

Automatische Detektion des dreidimensional geformten Graukartenpunktes/Mehrfarbenpunktes

Der dreidimensional geformte Graukartenpunkt/Mehrfarbenpunkt kann im 3D- Scandatensatz durch Abgleich der in der Software hinterlegten Geometrie des dreidimensional geformten Graukartenpunktes/Mehrfarbenpunktes (beispielsweise Halbkugel, Tetraeder, Pyramide etc.) (Sollwert) mit dem 3D-Scandatensatz (Istwert) detektiert werden. Als Berechnungsalgorithmus wird hierbei das sogenannte "Best-Fit- Verfahren" angewandt. Zur Ermittlung der Position des dreidimensional geformten Graukartenpunktes/Mehrfarbenpunktes wird durch iterative Prozesse die Standardabweichung stddev über den geringsten Abstand gebildet:

$$stddev = \sqrt{\frac{\sum_{i,j}^{n} \left[ (x_{1i} - x_{2j})^2 + (y_{1i} - y_{2j})^2 + (z_{1i} - z_{2j})^2 \right]}{n}}$$

Nach der Detektion des dreidimensional geformten Graukartenpunktes/Mehrfarbenpunktes im gescannten 3D-Datensatz können die den dreidimensionalen Punktwerten zugeordneten Farbwerte selektiert werden und der Abstand dieser Farbmesswerte (p) des n-dimensionalen Farbraumes zum Referenzfarbwert des Graukartenpunktes/Mehrfarbenpunktes (q) nach den oben beschriebenen Vorgehen berechnet werden.

BEZUGSZEICHENLISTE

1    Mehrfarbenpunkt
2    Farbe Weiß
3    Farbe Grau
4    Farbe Schwarz
5    Gingiva
6    Zahnreihen

**Patentansprüche**

1.  Verfahren zur digitalen Erfassung einer intraoralen Struktur durch Abtastung der Struktur mit einem Intraoralscanner, dessen Abtastwerte die räumliche Lage der Abtaststellen darstellende Ortsinformationsdaten und Farbwerte der Abtaststellen darstellende Farbinformationsdaten enthalten, wobei im Abtastbereich der Struktur mindestens eine Farbreferenzvorlage, die ein einem Referenzfarbwert eines Referenzfarbraums entsprechendes Referenzfarbfeld aufweist, angeordnet wird, und dass die abgetasteten Farbinformationsdaten entsprechend dem Abstand zwischen dem Referenzfarbwert und einem abgetasteten Farbwert des Referenzfarbfelds korrigiert werden, wobei aus der Menge der abgetasteten Farbwerte der Abtaststellen mindestens ein abgetasteter Farbwert des Farbreferenzfeldes durch einen softwareimplementierten Algorithmus selektiert wird, **dadurch gekennzeichnet, dass** durch den Algorithmus die euklidischen Abstände zwischen den abgetasteten Farbwerten der Abtaststellen und dem Referenzfarbwert des Referenzfarbfeldes berechnet werden und der für die Korrektur maßgebliche Abstand aufgrund einer Teilmenge der berechneten Abstände, die einer Minimalbedingung genügen, bestimmt wird.

2. Verfahren nach Anspruch 1, **dadurch gekennzeichnet, dass** das Referenzfarbfeld der Farbreferenzvorlage ein Graufeld aufweist.

3. Verfahren nach Anspruch 1 oder 2, **dadurch gekennzeichnet, dass** das Referenzfarbfeld der Farbreferenzvorlage ein Buntfeld aufweist.

4. Verfahren nach einem der Ansprüche 1 bis 3, **dadurch gekennzeichnet, dass** die korrigierten Abtastwerte für die Herstellung eines physischen Farbmodells oder einer dentalen Restauration, insbesondere einer Krone, Brücke oder Prothese, verwendet werden.

5. Verfahren nach Anspruch 4, **dadurch gekennzeichnet, dass** die Herstellung additiv, insbesondere mittels Multimaterial-3D-Druck, erfolgt.

6. Verfahren nach Anspruch 4, **dadurch gekennzeichnet, dass** die Herstellung subtraktiv, insbesondere durch Fräsen, Pressen, Gießen oder Tiefziehen, erfolgt.

7. Verfahren nach Anspruch 1, **dadurch gekennzeichnet, dass** der maßgebliche Abstand als Mittelwert über Abstände der Teilmenge berechnet wird.

8. Verfahren nach Anspruch 1, **dadurch gekennzeichnet, dass** durch den Algorithmus aus der Menge der abgetasteten Ortsinformationsdaten in einem Best-Fit-Verfahren Ortsinformationsdaten des Referenzfarbfeldes ermittelt werden und der für die Korrektur maßgebliche Abstand aufgrund den ermittelten Ortsinformationsdaten zugeordneter Farbwerte bestimmt wird.

9. Verfahren nach Anspruch 1, **dadurch gekennzeichnet, dass** aus der Menge der abgetasteten Ortsinformationsdaten durch einen Bildsegmentierungsalgorithmus Ortsinformationsdaten des Referenzfarbfeldes ermittelt werden und der für die Korrektur maßgebliche Abstand aufgrund den ermittelten Ortsinformationsdaten zugeordneter Farbwerte bestimmt wird.

10. System zur Durchführung des Verfahrens nach einem der Ansprüche 1 bis 9, mit einem Intraoralscanner, dessen Abtastwerte die räumliche Lage der Abtaststellen darstellende Ortsinformationsdaten und Farbwerte der Abtaststellen darstellende Farbinformationsdaten enthalten, mit einer Farbreferenzvorlage, die ein einem Referenzfarbwert eines Referenzfarbraums entsprechendes Referenzfarbfeld aufweist und mit einer Einrichtung zum Korrigieren der abgetasteten Farbinformationsdaten entsprechend dem Abstand zwischen dem Referenzfarbwert und einem abgetasteten Farbwert des Referenzfarbfeldes und das System angepasst ist, aus der Menge der abgetasteten Farbwerte der Abtaststellen mindestens einen abgetasteten Farbwert des Farbreferenzfeldes durch einen softwareimplementierten Algorithmus zu selektieren, **dadurch gekennzeichnet, dass** das System angepasst ist, durch den Algorithmus die euklidischen Abstände zwischen den abgetasteten Farbwerten der Abtaststellen und dem Referenzfarbwert des Referenzfarbfeldes zu berechnen und den für die Korrektur maßgeblichen Abstand aufgrund einer Teilmenge der berechneten Abstände, die einer Minimalbedingung genügen, zu bestimmen.

11. System nach Anspruch 10, mit einem von den korrigierten Abtastwerten gesteuerten Multimaterial-3D-Drucker.

## Claims

1. A method for digitally detecting an intraoral structure by scanning the structure with an intraoral scanner, the scanned values of which contain location information data representing the spatial position of the scanning points and color information data representing color values of the scanning points, wherein at least one color reference template having a reference color field corresponding to a reference color value of a reference color space is arranged in the scanning area of the structure, and wherein the scanned color information data is corrected according to the distance between the reference color value and a scanned color value of the reference color field, wherein at least one scanned color value of the color reference field is selected from the set of scanned color values of the scanning points by a software-implemented algorithm, **characterized in that** the Euclidean distances between the scanned color values of the scanning points and the reference color value of the reference color field are calculated by the algorithm and the distance relevant for the correction is determined on the basis of a subset of the calculated distances which satisfy a minimum condition.

2. The method according to claim 1, **characterized in that** the reference color field of the color reference template comprises a gray field.

3. The method according to claim 1 or 2, **characterized in that** the reference color field of the color reference template comprises a chromatic field.

4. The method according to one of the claims 1 to 3, **characterized in that** the corrected scanned values are used for the production of a physical color model or a dental restoration, in particular a crown, bridge or prosthesis.

5. The method according to claim 4, **characterized in that** the production is carried out additively, in particular by means of multi-material 3D printing.

6. The method according to claim 4, **characterized in that** the production is carried out subtractively, in particular by milling, pressing, casting or deep drawing.

7. The method according to claim 1, **characterized in that** the relevant distance is calculated as the mean value by means of distances of the subset.

8. The method according to claim 1, **characterized in that** location information data of the reference color field is determined by the algorithm from the set of the scanned location information data in a best-fit method and the distance relevant for the correction is determined on the basis of the color values assigned to the determined location information data.

9. The method according to claim 1, **characterized in that** location information data of the reference color field is determined from the set of scanned location information data by an image segmentation algorithm and the distance relevant for the correction is determined on the basis of the color values assigned to the determined location information data.

10. A system for carrying out the method according to any one of claims 1 to 9, comprising an intraoral scanner, the scanned values of which contain location information data representing the spatial position of the scanning points and color information data representing color values of the scanning points, comprising a color reference template having a reference color field corresponding to a reference color value of a reference color space, and comprising means for correcting the scanned color information data according to the distance between the reference color value and a scanned color value of the reference color field, and wherein the system is configured to select at least one scanned color value of the color reference field from the set of scanned color values of the scanning points by a software-implemented algorithm, **characterized in that** the system is configured to calculate the Euclidean distances between the scanned color values of the scanning points and the reference color value of the reference color field by the algorithm and to determine the distance relevant for the correction on the basis of a subset of the calculated distances which satisfy a minimum condition.

11. The system according to claim 10, comprising a multimaterial 3D printer controlled by the corrected scanned values.

**Revendications**

1. Procédé d'acquisition numérique d'une structure intra-orale par balayage de la structure à l'aide d'un scanner intra-oral, dont les valeurs de balayage contiennent des données d'information de localisation représentant la position spatiale des points de balayage et des données d'information de couleur représentant les valeurs de couleur des points de balayage, où au moins un modèle de référence de couleur, qui présente un champ de couleur de référence correspondant à une valeur de couleur de référence d'un espace de couleur de référence, est disposé dans la zone de balayage de la structure, et en ce que les données d'information de couleur numérisées sont corrigées en fonction de la distance entre la valeur de couleur de référence et une valeur de couleur numérisée du champ de couleur de référence, où, à partir de l'ensemble des valeurs de couleur numérisées des points de numérisation, au moins une valeur de couleur numérisée du champ de référence de couleur est sélectionnée par un algorithme implémenté par logiciel, **caractérisé en ce que** l'algorithme calcule les distances euclidiennes entre les valeurs de couleur échantillonnées des points d'échantillonnage et la valeur de couleur de référence de la zone de couleur de référence, et la distance déterminante pour la correction est déterminée sur la base d'un sous-ensemble des distances calculées qui satisfont à une condition minimale.

2. Procédé selon la revendication 1, **caractérisé en ce que** le champ de couleur de référence du modèle de référence de couleur présente un champ gris.

3. Procédé selon la revendication 1 ou 2, **caractérisé en ce que** le champ de couleur de référence du modèle de référence de couleur présente un champ coloré.

4. Procédé selon l'une des revendications 1 à 3, **caractérisé en ce que** les valeurs d'échantillonnage corrigées sont utilisées pour la fabrication d'un modèle physique en couleur ou d'une restauration dentaire, en particulier d'une couronne, d'un bridge ou d'une prothèse.

5. Procédé selon la revendication 4, **caractérisé en ce que** la fabrication est réalisée de manière additive, en particulier au moyen d'une impression 3D multi-matériaux.

6. Procédé selon la revendication 4, **caractérisé en ce que** la fabrication s'effectue de manière soustractive, en particulier par fraisage, pressage, moulage ou emboutissage.

7. Procédé selon la revendication 1, **caractérisé en ce que** la distance déterminante est calculée comme une valeur moyenne des distances du sous-ensemble.

8. Procédé selon la revendication 1, **caractérisé en ce que** l'algorithme détermine les données d'information de localisation du champ de couleur de référence à partir de l'ensemble des données d'information de localisation échantillonnées dans un processus de meilleur ajustement, et que la distance déterminante pour la correction est déterminée sur la base des valeurs de couleur attribuées aux données d'information de localisation déterminées.

9. Procédé selon la revendication 1, **caractérisé en ce que** les données d'information de localisation du champ de couleur de référence sont déterminées à partir de l'ensemble des données d'information de localisation échantillonnées à l'aide d'un algorithme de segmentation d'image et que la distance déterminante pour la correction est déterminée sur la base des valeurs de couleur attribuées aux données d'information de localisation déterminées.

10. Système pour la mise en œuvre du procédé selon l'une des revendications 1 à 9, comprenant un scanner intra-oral dont les valeurs de balayage contiennent des données d'information de localisation représentant la position spatiale des points de balayage et des données d'information de couleur représentant les valeurs de couleur des points de balayage, avec un modèle de référence de couleur qui présente un champ de couleur de référence correspondant à une valeur de couleur de référence d'un espace de couleur de référence et avec un dispositif pour corriger les données d'information de couleur échantillonnées en fonction de la distance entre la valeur de couleur de référence et une valeur de couleur échantillonnée du champ de couleur de référence, et le système est adapté sélectionner, à partir de l'ensemble des valeurs de couleur échantillonnées des points d'échantillonnage, au moins une valeur de couleur échantillonnée du champ de référence de couleur à l'aide d'un algorithme implémenté par logiciel, **caractérisé en ce que** le système est adapté pour calculer, à l'aide de l'algorithme, les distances euclidiennes entre les valeurs de couleur échantillonnées des points d'échantillonnage et la valeur de couleur de référence du champ de couleur de référence, et pour déterminer la distance déterminante pour la correction sur la base d'un sous-ensemble des distances calculées qui satisfont à une condition minimale.

11. Système selon la revendication 10, avec une imprimante 3D multi-matériaux commandée par les valeurs d'échantillonnage corrigées.

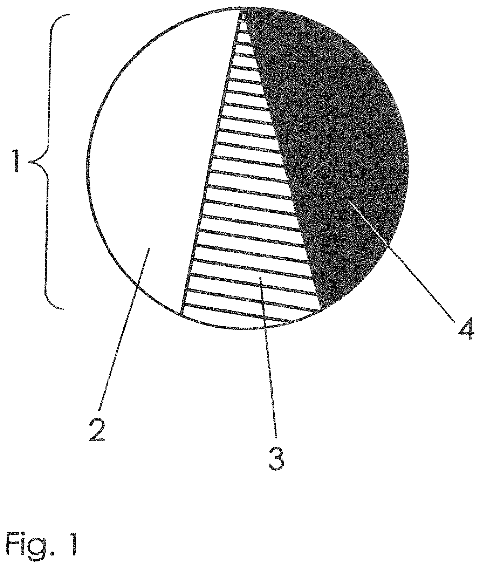

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2017252135 A2
- US 2008094631 A1
- DE 102006061893 B3